# EUROPÄISCHE PATENTSCHRIFT

(11) **EP 1 180 372 B1**
(45) Veröffentlichungstag und Bekanntmachung des Hinweises auf die Patenterteilung: **02.01.2004**
(21) Anmeldenummer: 01119821.5
(22) Anmeldetag: 16.08.2001
(51) Int. Cl.: A61L 29/04, A61L 33/06

(54) **Dialyseset aus PVC-freien, transparenten, blutkompatiblen und lösemittelverklebbaren Polyolefinen**
Dialysis device of PVC-free, transparent, blood-compatible polyolefins which can be assembled in the presence of a solvent
Emsemble de dialyse à base de polyoléfines poisseuses exemptes de PVC, transparentes et compatibles avec du sang

(30) Priorität: 17.08.2000 DE 10040266
(43) Veröffentlichungstag der Anmeldung: 20.02.2002
(73) Patentinhaber: REHAU AG + Co, 95111 Rehau (DE)
(72) Erfinder: Kühlein, Georg, c/o Rehau AG + Co., 95111 Rehau (DE)

(56) Entgegenhaltungen:
- EP-A- 0 518 061
- DE-A- 3 914 998
- DE-A- 4 219 071
- DE-A- 19 650 673
- DE-A- 19 719 593
- US-A- 5 562 127

## Beschreibung

Stand der Technik bei der Dialyse sind Sets aus PVC, die unter anderem über die nötige Transparenz und Blutkompatibilität verfügen, weiterhin in der Herstellung wegen der günstigen Materialkosten und ihrer guten und sicheren Lösemittelverklebbarkeit besonders wirtschaftlich sind.
PVC weist bekanntermaßen jedoch auch eine Reihe von Nachteilen auf. Zu nennen ist hierbei beispielsweise die Weichmachermigration: Da die herkömmlichen Weichmacher fettlöslich sind, werden sie in Folge des Fettgehaltes des Blutes ausgewaschen und transportieren als Leitsubstanzen weitere Bestandteile mit allergenem Potential aus den Werkstoffen in das Patientenblut. Nachteilig bei PVC ist auch, dass die Entsorgung durch Verbrennung wegen der Entstehung von Chlorwasserstoff und der möglichen Entstehung von Dioxinen nicht unproblematisch ist.

Stand der Technik bei Infusion/Transfusion sind unter anderem Sets aus Polystyrolpolymeren, wie beispielsweise in DE 39 14 998 beschrieben oder aus Polyolefinen, wie DE 42 19 071 lehrt. Dass diese Werkstoffe bisher nicht in der Dialyse eingesetzt wurden, liegt vor allem an deren völlig unzureichender Blutkompatibilität. Weiterhin: Die Polystyrol-Polymeren sind in ihrer Spannungsrissbeständigkeit unbefriedigend, die Polyolefin-basierenden Werkstoffe wenig transparent, und wenn sie weichmachende, niedermolekulare Komponenten enthalten sind sie nicht migrationsbeständig. Insbesondere aber sind sie nicht, wie PVC, verklebbar, sondern nur anlösbar, so dass lediglich eine Adhäsionsfixierung über Steckkonfektion möglich ist. Nachteilig ist hierbei für die Dialyse, dass, anders als bei der Infusion, beträchtliche Drücke im Leitungssystem auftreten, so dass die beschriebene Adhäsionsfixierung mit erheblichen Risiken für den Patienten verbunden ist.

DE 197 19 593 beschreibt Polymermischungen aus Polyolefinen und statistischen Copolymeren aus styrolischen Segmenten und Kohlenwasserstoffsegmenten, wobei der Anteil der statistischen, styrolischen Segmente enthaltenen Copolymeren zwischen 5 und 95 Gewichtsprozent, bevorzugt zwischen 20 und 70 Gewichtsprozent liegt.
Verwendung finden derartige Polymermischungen für die Herstellung medizinischer Schläuche, Getränkeschläuche, Folienschläuche usw. Diese Polymermischungen sind zwar dampfsterilisierbar, jedoch nicht blutkompatibel und - im bevorzugten Bereich - nicht lösemittelverklebbar.

***EP 0518 061 A lehrt medizinische Arbeitsmittel, wie Katheter, Schläuche, Behälter und Formteile aus PVC, die eine besondere Ausstattung zur Erhöhung der Blutkompatibilität aufweisen. Die verbesserte Blutkompatibilität erhält man durch Einmischen einer definierte Menge von Seltenerdcarboxylaten in das Ausgangspolymer vor der eigentlichen Formgebung.***
***Diese medizinischen Arbeitsmittel weisen jedoch die für PVC-Artikel üblichen Nachteile auf, wie Weichmachermigration oder Probleme bei der Entsorgung durch Verbrennung.***

***US 5 562 127 A offenbart flexible, chlorfreie Schläuche für die Medizintechnik und ein Verfahren zu deren Herstellung.***
***Der Schlauch ist aufgebaut mindestens aus einer Innenschicht, dessen Polymer einen Young-Modul zwischen 2 und 60 MPa aufweist, und einer Außenschicht, dessen Polymer nicht mehr als das 15-fache des Moduls der Innenschicht aufweist.***
***Die Schichtpolymere bestehen aus Polyolefinen oder Copolymeren aus Polyolefinen mit substituierten Polyolefinen.***
***Nachteilig ist hierbei, dass es sich hierbei um Schläuche handelt, die nicht transparent sind, für die Dialyse mangels Blutkompatibilität nicht geeignet sind und die sich gemäß der bei PVC üblichen Konfektionstechnik nicht verkleben lassen*.**

Für die Konfektion kompletter Dialysesets ist nach wie vor PVC der Stand der Technik. Daher wurde in der Medizintechnik-Industrie in den letzten Jahren in vollautomatische Großanlagen mit einer Ausbringung von ca 6.000 Dialysesets pro Tag, investiert. Diese sind ausschließlich auf die Verarbeitung von mit PVC verklebbaren Materialien ausgelegt.
Bisher war es nicht möglich, unter Beibehaltung der herkömmlichen Accessoires, wie Tropfenkammer, Konnektoren, Schlauchklemmen usw auf diesen Anlagen beispielsweise Schläuche aus Polyolefinen zu verarbeiten. Das liegt an den völlig unterschiedlichen mechanischen Eigenschaften der herkömmlichen Polyolefinrezepturen. Zu nennen wäre hier beispielsweise die geforderte Weichheit in Verbindung mit hohem Rückstellvermögen und die schon erwähnte Tatsache, dass sie nicht lösemittelverklebbar sind.

Die Erfindung und die Überlegungen, die zur Erfindung geführt haben, sollen nachfolgend näher erläutert werden.

Im Unterschied zur Infusion oder Transfusion von wässrigen Lösungen werden an Systeme, die in der Dialyse eingesetzt werden sollen, besonders strenge Anforderungen gestellt:
a) Möglichst hohe Transparenz > 70 %, um evtl. Luftbläschen zu erkennen
b) Optimale Blutkompatibilität
   Diese lässt sich anhand der Bestimmung der Thrombogenität der Werkstoffe nach dem Resonanzthropografier-Verfahren aufzeigen. Beispielsweise liegt die Plättchenamplitude im Fall von PVC bei 2,4, im Falle von Polypropylen bei 12.
c) Die Polyolefine müssen über eine weite Bandbreite in ihrer Härte variabel einstellbar sein können.
   Beispielsweise müssen Fistelnadelschläuche über eine Shorehärte von A 60 verfügen, also extrem weich sein, andererseits wird für die Leitungsschläuche eine hohe Knickstabilität gefordert, von den Pumpschläuchen ist eine hohe Weichheit bei gleichzeitig hohem Rückstellvermögen und hoher Abriebfestigkeit gefordert. Letztere Eigenschaften sind notwendig, um die Pumpfunktion zu gewährleisten und die Patienten nicht durch abgeriebene Partikel, die Gefäßverschlüsse verursachen können, zu gefährden.
d) Zuverlässige, wirtschaftliche Verbindungstechnik wie bei PVC durch problemlose Verklebbarkeit mit Lösemitteln.
e) Kein Gehalt an Weichmachern bzw keine Weichmachermigration
f) Sterilisierbar mit Ethylenoxid oder Gammastrahlen.

Aus den genannten Anforderungen ergibt sich eine Kombination von Materialeigenschaften, die einander auszuschließen scheinen. Beispielsweise verschlechtern für gewöhnlich weichmachende Komponenten zu Polyolefinen die Transparenz. Oder die Additive, welche die Verklebbarkeit erhöhen, verringern gleichzeitig die Stabilität der Mischung nach der Sterilisation mit Gammastrahlen. Oder die Additive, welche die Blutkompatibilität erhöhen, führen zu einer starken Eintrübung.
Durch die theoretische Kombination des Standes der Technik wird also nicht nahe gelegt, dass sich in der Summierung ein für den vorgesehenen Zweck gut geeigneter Werkstoff ergeben könnte.

Aufgabe der Erfindung war es, die Nachteile des Standes der Technik zu überwinden und einen Schlauch für die Dialyse zur Verfügung zu stellen, der wirtschaftlich herstellbar und halogenfrei ist, insbesondere blutkompatibel, weich, aber weichmacherfrei, mit den bekannten Konfektionstechniken, für PVC verarbeitbar und mit Gammastrahlen oder Ethylenoxid sterilisierbar ist.

Erfindungsgemäß konnte die Aufgabe überraschenderweise durch einen Schlauch für den Einsatz in der Dialyse mit den Merkmalen des Anspruches 1 gelöst werden.

Die Erfindung ermöglicht also den völligen Verzicht auf weichgemachtes PVC und erschließt durch die blutkompatiblen und verklebbaren Formulierungen erstmals den Polyolefinen die Anwendung in der Dialyse.

Nachfolgend soll die Erfindung näher beschrieben werden und dabei auf die für Dialysesets wichtigen Werkstoffeigenschaften eingegangen werden. Dabei handelt es sich um die Eigenschaften Verbindungsfestigkeit, Transparenz, Blutkompatibilität, Rückstellvermögen und Weichmachermigration.

Es ist bekannt, dass beim Mischen transparenter Polymere die Klarsichtigkeit der Endprodukte verloren geht und sich diese Teile opak bis trüb darstellen. Die erfindungsgemäße Kombination der Rezepturbestandteile führt dem gegenüber zu Produkten, die sich in ihrer Transparenz von PVC zwar unterscheiden, wobei allerdings sichergestellt ist, dass die in einer Dialyseapparatur installierten Überwachungsdetektoren evtl. Luftbläschen im System fehlerfrei erkennen und Alarm auslösen können.

***Die erfindungsgemäße Werkstoff ist zusammensetzt aus einer polyolefinische Komponente, umfassend Polypropylen- Homo- oder -Copolymere und*/*oder Mischungen davon und statistische Copolymere, bestehend aus styrolischen Segmenten und Kohlenwasserstoffsegmenten, wobei der Anteil an styrolischen Segmenten im statistischen Copolymer 70 - 90 Gew.% beträgt, und einer die Blutkompatibilität fördernde Komponente, die zusammengesetzt ist aus N,N'-Diacyl-Ethylendiamin und einem oder mehreren Stearaten der seltenen Erden.***

Der entscheidende Vorteil, der den erfindungsgemäßen polyolefinischen Rezepturen den Zugang zu den Einwegartikeln der Dialysetechnik ermöglicht, ist die durch die Erfindung erreichte Verklebbarkeit. Da es sich bei dem Dialyseset um ein Leitungssystem handelt, das für den extrakorporalen Kreislauf benötigt wird, sind, der Sicherheit der Patienten wegen, absolut zuverlässige Klebestellen erforderlich, und Leckagen, gleich welcher Art, zwingend zu vermeiden. Diese Sicherheit ist über eine mehrstündige Behandlungsdauer und bei Blutdruckniveau zu gewährleisten. Diese geforderte Zuverlässigkeit wurde bislang ausschließlich über den Werkstoff PVC erreicht.

Verklebt man PVC mit PVC mittels Tetrahydrofuran oder Cyklohexanon oder einer Mischung aus beiden, werden im Zugversuch bei einer Prüfgeschwindigkeit von 100 mm pro Minute Verbindungsfestigkeiten von 270 N erreicht. Dieser Wert liegt bereits im Bereich der reinen Materialfestigkeit von PVC.
Die bisher in der Literatur beschriebenen Polyolefinverklebungen beruhen auf einem Anlösen des nicht-olefinischen Gegenstücks. Die Oberfläche dieses lösungsmittelempfindlichen Teiles passt sich unter Lösemitteleinwirkung dem olefinischen Gegenüber an und bildet dabei für Verbindungen, die nicht druckbelastet sind, ausreichende Adhäsionsfestigkeiten aus. Der Unterschied zwischen einer solchen Adhäsionsverbindungen und einer, mit einer Kaltverschweißung vergleichbaren Verklebung gemäß der Erfindung, wird deutlich, wenn beispielsweise ein Schlauch mit Druck beaufschlagt wird. Ein auf einem Konnektor aufgeschobener, herkömmlicher polyolefinischer Schlauch wird sich weiten und problemlos vom Konnektor abziehen lassen. Der nach dieser Erfindung hergestellte Schlauch aus einer polyolefinischen Legierung jedoch lässt sich nicht lösen, weil er durch die Lösungsmittelverklebung mit dem Formteil fest verbunden ist.
Die Lösungsmitttelverklebung der erfindungsgemäßen polyolefinischen Legierung weist mit 250 N im Mittel Verbindungsfestigkeiten auf, die mit PVC vergleichbar sind.

Ein weiteres Hindernis, polyolefinische Werkstoffe in der Medizin zu verwenden, war bisher die mangelnde Blutkompatibilität.

Ein typisches Beispiel dafür ist die Tatsache, dass die zunächst weit verbreiteten polyolefinisches Venenkatheter aus Gründen mangelnder Blutkompatibilität durch solche aus - wesentlich teurerem - TPU wieder abgelöst wurden.
Die Blutkompatibilität wird üblicherweise in Maßzahlen ausgedrückt. Die Maßzahlen sind dabei die Verhältniswerte von nativem und behandeltem Blut. Als Testverfahren hat sich dabei die sog. Resonanzthrombografie bewährt. Natives Blut hat die Maßzahl 1, Weich-PVC ca. 2,5, Silikon ca. 6, TPU liegt knapp über 1, Polyolefine liegen bei etwa 12.

Die Erfindung ermöglichst es nunmehr, Polyolefine in ihrer Blutverträglichkeit an die von PVC heranzuführen. Dies geschieht durch Einlegieren von geeigneten Copolymeren aus styrolischen Segmenten und Kohlenwasserstoffsegmenten, wobei der Anteil an styrolischen Segmenten erfindungsgemäß möglichst hoch sein soll, in Kombination mit ausgewählten, die Blutverträglichkeit erhöhenden Additiven, welche offenbar in den erfindungsgemäßen Formulierungen synergistisch wirken und die Gerinnungskaskade wirksam blockieren.

Zu Erläuterung seien - am Beispiel eines Dialyseschlauches der Shorehärte A 80 - die wichtigsten Eigenschaften der Erfindung denen des Standes der Technik gegenübergestellt. Die erfindungsgemäße Rezeptur im vorliegenden Beispiel umfasst folgende Bestandteile:

| | |
|---|---|
| Gewichtsteile Polypropylen-Copolymer | 48 % |
| statistisches Copolymer 90 Gew.% an styrolischen Segmenten | 50 % |
| Gewichtsteile N-N'-Diacyl-Ethylendiamin | 1 % |
| Gewichtsteile Neodymstearat | 1 % |

Als Referenz dient ein Dialyseset aus PVC und ein Infusionsset nach DE 42 19 071.

| **Werkstoff** | **Transparenz %** | **Weichmachermigration** | **Blutkompatibilität** | **Verbindungsfestigkeit** | **Rückstellvermögen** |
|---|---|---|---|---|---|
| PVC | 95 | 2,83 µg | 2, 4 | 270 N | 15% |
| DE 4219071 | 70 | keine | 12, 5 | 65 N | 50% |
| Erfindung | 80 | keine | 3, 3 | 250 N | 40% |

- Weichmachermigration: der Zahlenwert bezieht sich auf die Weichmacheraufnahme in einem ml Patientenblut über einen Dialysecyclus von 3 Stunden
- Verbindungsfestigkeit: Ausreißkraft eines Konnektors aus einem Schlauch ( Zugversuch)
- Rückstellvermögen: gemessen als Druckverformungsrest bei 20 °C

Nachfolgend sei nun an einem Ausführungsbeispiel die erfindungsgemäße Vorrichtung zum Einsatz in der Dialyse näher beschrieben.

### Zunächst einige Vorbemerkungen:

Ein standardmäßiges Dialyseset besteht aus einer venösen und einer arteriellen Linie.

Die heute bekannte Mannigfaltigkeit der Systeme ist zum einen auf die unterschiedlichen Dialysemaschinen, und zum anderen auf anwendungsspezifische Abhängigkeiten zurückzuführen. Dialysesets bestehen heute ausschließlich aus Weich-PVC-Schläuchen unterschiedlicher Abmessung, kombiniert mit diversen Accessoires, aus Hart-PVC, PC, PMMA und Polymeren oder Copolymeren des Styrols - in Anhängigkeit von der Dialysemaschine und den spezifischen Erfordernissen.
Die Schläuche und auch die diversen Spritzgussteile sind härtemäßig und maßlich weitgehend standardisiert, um eine vollautomatische Fertigung zu ermöglichen.
Die Dialysesets werden heute vollautomatisch, halbautomatisch oder händisch konfektioniert. Allen Verfahren gemeinsam ist die vorangestellte Fertigung von Baugruppen.

In Deutschland werden Dialysesets zurzeit in etwa 130 gängigen Varianten eingesetzt.
Im nachfolgenden Ausführungsbeispiel wird ein typisches Dialyseset beschrieben. Es besteht erfindungsgemäß aus halogenfreien Werkstoffen, wobei die arterielle Linie 27 Einzelteile, das venöse Gegenstück dazu 18 Einzelteile umfasst.

### Arterielle Linie:

1. Verschlußkappe
2. Dialysatorkonnektor
3. Schlauch 4,1 x 6,8 mm, 600 mm lang
4. Tropfenkammer
5. Luer-Lock-Anschluss weiblich für Schlauchaußendurchmesser 4,1 mm
6. Schlauch 3,0 x 4,1 mm, 100 mm lang
7. Verschlussklemme
8. Schutzkappe für den Luer-Lock-Anschluss weiblich
9. Schlauch 4,1 x 6,8 mm, 600 mm lang
10. Pumpenkonnektor, auf Pumpschlauchgröße Außendurchmesser 12,2 mm
11. Schlauch als Heparinleitung 1,0 x 2,0 mm, 1.000 mm lang
12. Luer-Lock-Anschluss weiblich
13. Verschlussklemme
14. Schutzkappe für den Luer-Lock-Anschluss weiblich
15. Pumpenkonnektor, auf Pumpschlauchgröße Außendurchmesser 12,2 mm
16. Pumpschlauch Außendurchmesser 12,2 mm, 250 mm lang
17. Schlauch 4,1 x 6,8 mm, 60 mm lang
18. Unterdruckkissen
19. Luer-Lock-Anschluss weiblich für Schlauch-Außendurchmesser 4,1mm
20. Schutzkappe für den Luer-Lock-Anschluss weiblich
21. Schlauch 3,0 x 4,1 mm, 100 mm lang
22. Schlauch 4,1 x 6,8 mm, 250 mm lang
23. Zuspritzteil
24. Schlauch 4,1 x 6,8 mm, 1.750 mm lang
25. Verschlussklemme
26. Fistelnadel-Konnektor
27. Fistelnadel-Kappe

### Venöse Linie:

1. Verschlußkappe für den Dialysator-Konnektor
2. Diatysator-Konnektor
3. Schlauch 4,1 x 6,8 mm, 1.000 mm lang
4. Verschlußkappe für Luer-Lock-Anschluss
5. Luer-Lock-Anschluss weiblich für Schlauch 3,0 x 4,1 mm
6. Verschlussklemme
7. Schlauch 3,0 x 4,1 mm, 80 mm lang
8. Schutzkappe für den Luer-Lock-Anschluss weiblich
9. Filter
10. Schutzkappe für den Filter
11. Schlauch 4,1 x 5,5 mm, 250 mm lang
12. Tropfkammer mit Blutfilter
13. Schlauch 4,1 x 6,8 mm, 250 mm lang
14. Zuspritzteil
15. Schlauch 4,1 x 6,8 mm, 1750 mm lang
16. Verschlussklemme
17. Fistelnadel-Konnektor
18. Fistelnadel-Konnektorkappe.

Die Montage des kompletten Dialysesets beginnt mit der Vorfertigung erster Komponenten.
- Bereits vorgefertigt angeliefert sind in der Regel die Luer-Lock-Anschtüsse, die Dialysatoren-Konnektoren, die Fistelnadel-Konnektoren und die Filter, jeweils mit den Schutzkappen.
- Bei der venösen Tropfkammer sind die Blutfilter bereits integriert.
- Die Schläuche werden in der Regel als Rollenware, die Accessoires als Schüttgut angeliefert.
- Bei der automatischen Konfektion werden dabei die jeweiligen Klebestellen der zu verbindenden Einzelteile über eine Düse mit Tetrahydrofuran angenebelt und dann unter Druck miteinander verbunden.

Tropfkammer und Tropfkammerdeckel werden in einem ersten Schritt verklebt. Vorgefertigt werden weiter die Schläuche 3,0 x 4,1 mm mit den Luer-Lock-Anschlüssen, dazu wird einseitig der Luer-Lock-Anschluss weiblich mit der Schutzkappe aufgeklebt und die Schlauchklemme aufgefädelt. Ebenso vorbereitet wird der Heparinschlauch mit der Abmessung 1,0 x 2,0 mm und der Länge 1.000 mm. Die Verschlussklemme wird aufgefädelt und am Ende der Luer-Lock-Anschluss mit der Schutzkappe angeklebt. In die Vorkonfektion geht auch der Pumpschlauch mit den Außendurchmessern 12,2 mm, an dessen beiden Enden die Pumpschlauchkonnektoren angeklebt werden. Analog wird beim Schlauch 4,1 x 6,8 mm der Fixlänge 600 mm der Dialysekonnektor mit Kappe aufgeklebt. An den Schlauch 4,1 x 6,8 mm der Länge 1.750 mm wird endseitig der Fistelnadelkonnektor mit Schutzkappe angeklebt. Anschließend wird die Schlauchklemme aufgefädelt und das Zuspritzteil eingeklebt. Die Leitung wird durch Einkleben eines weiteren Schlauchabschnittes der Abmessung 4,1 x 6,8 mm, Länge 250 mm, verlängert.

In der Folge werden nun aus den Komponenten die Baugruppen komplettiert: In den Tropfkammerdeckel wird die Zuspritzleitung eingeklebt, die aus dem Luer-Lock-Anschluss weiblich mit Schutzkappe, Schlauch und der Schlauchklemme besteht. In den Pumpenschlauch-Konnektor wird an den seitlichen Abgang die Heparinleitung mit Schlauchklemme und Luer-Lock-Anschluss mit Schutzkappe eingeklebt. Analog wird der Schlauch 3,0 x 4,1 mm mit der aufgefädelten Klemme und dem Luer-Lock-Anschluss weiblich mit Schutzkappe in den Abgang des Unterdruckkissens geklebt.

Diese nunmehr hergestellten Baugruppen und vorkonfektionierten Teile erlauben jetzt die Endkonfektion der arteriellen Dialyselinie.

Dazu wird nunmehr der Schlauchabschnitt mit dem Dialysekonnektor in den Boden der Tropfkammer eingeklebt. In den Deckel eingeklebt wird der Schlauch 4,1 x 6,8 mm in der Länge 600 mm und der Schlauch mit Klemme, Luer-Lock-Anschluss weiblich und Schutzkappe. Das Ende dieses Schlauches 4,1 x 6,8 mm am Tropfkammerdeckel wird nunmehr mit dem Pumpschlauchsegment auf der Seite mit dem Zugang über die Heparinleitung verklebt. Auf der anderen Seite des Pumpschlauches wird ein Schlauchabschnitt der Abmessung 4,1 x 6,8 mm mit einer Länge von 60 mm eingeklebt. Über Verklebung erfolgt jetzt die Kombination mit dem Unterdruckkissen mit der Zuspritzleitung. Der Schlauch 4,1 x 6,8 mm, mit eingeklebtem Zuspritzteil, einer aufgefädelten Klemme und dem Fistelnadel-Konnektor mit Kappe komplettiert letztlich die arterielle Linie.

Die venöse Linie wird vom Prinzip her analog vorgefertigt und endkonfektioniert.

Vor der Endverpackung werden die venösen und arteriellen Linien über eine 100 %-Kontrolle auf Dichtigkeit geprüft.
Die beiden Linien werden nun zu handlichen Bestecken aufgerollt. Bänder sichern die Systeme gegen das Verrutschen der Lagen.
Die so hergestellten Dialysesets werden in sog. Peelbags pärchenweise eingesiegelt und in Kartons endverpackt.
Die nachfolgende Sterilisation erfolgt mittels Ethylenoxid.

## Patentansprüche

1. Schläuche für den Einsatz in der Dialyse aus polymeren, halogenfreien und weichmacherfreien Werkstoffen, umfassend ein arterielles und ein venöses System,
**dadurch gekennzeichnet, dass**
die wesentlichen, mit Blut in Kontakt kommenden Bestandteile aus einem Werkstoff bestehen, der über eine Verbindungsfestigkeit von mindestens 200 N verfügt, dessen Maßzahl für die Blutkompatibilität kleiner als 5 ist und umfasst
- eine polyolefinische Komponente, zusammengesetzt aus Polypropylen-Homound /oder -Copolymeren oder Mischungen davon, sowie statistischen Copolymeren, bestehend aus styrolischen Segmenten und Kohlenwasserstoffsegmenten, wobei der Anteil an styrolischen Segmenten im statistischen Copolymer zwischen 30 und 90 Gewichtsprozent, bevorzugt 70 - 90 Gewichtsprozent beträgt
- eine die Blutkompatibilität fördernde Komponente, die zusammengesetzt ist aus N,N'-Diacyl-Ethylendiamin und einem oder mehreren Stearaten der seltenen Erden.

2. Schläuche nach Anspruch 1, **dadurch gekennzeichnet, dass** als weitere Komponenten mit den statistischen, styrolischen Copolymeren verträgliche Öle, sowie Verarbeitungshilfsmittel, umfassend Gleitmittel und/oder Stabilisatoren, enthalten sind.

## Claims

1. Tubing for use in dialysis, made of halogen-free and plasticiser-free polymer materials, comprising an arterial system and a venous system,
**characterised in that**
the essential blood-contacting components consist of a material having a connection strength of at least 200 N and a blood compatibility value less than 5, and comprising
- a polyolefin component, composed of polypropylene homopolymers and/or polypropylene copolymers or mixtures thereof, and statistical copolymers, consisting of styrene segments and hydrocarbon segments, the proportion of styrene segments in the statistical copolymer being between 30 and 90 percent by weight, preferably 70 to 90 percent by weight;
- a blood-compatibility-promoting component composed of N,N'-diacyl-ethylenediamine and one or more rare earth metal stearates.

2. Tubing as defined in claim 1, **characterised in that** further components include oils which are compatible with the statistical styrene copolymers, and also processing auxiliaries, including lubricants and/or stabilisers.

## Revendications

1. Tuyaux de dialyse, en matières polymères, sans halogènes ni plastifiants, recouvrant un système artériel et veineux,
**caractérisés en ce que**
les principaux composants entrant en contact avec le sang sont constitués d'une matière disposant d'une stabilité de liaison d'au minimum 200 N, dont le chiffre d'indice pour la compatibilité avec le sang est inférieur à 5 et qui comprend
- un élément polyoléfinique, qui se compose d'homopolymères et/ou de copolymères de polypropylène ou de mélanges de ceux-ci ainsi que de copolymères statistiques constitués de segments styréniques et de segments hydrocarbonés, la proportion de segments styréniques dans le copolymère statique se situant entre 30 et 90 en pourcentage pondéral, de préférence entre 70 et 90
- un élément constituant favorisant la compatibilité avec le sang, qui se compose de N,N'-diacyle éthylène diamine et d'un ou plusieurs stéarates de terres rares.

2. Tuyaux selon revendication 1, **caractérisés en ce qu'**ils contiennent comme autres composants, des huiles compatibles avec les copolymères styréniques statistiques ainsi que des adjuvants de mise en oeuvre englobant des lubrifiants et/ou des stabilisants.
